# EUROPÄISCHE PATENTSCHRIFT

(11) **EP 2 146 588 B1**
(45) Veröffentlichungstag und Bekanntmachung des Hinweises auf die Patenterteilung: **28.11.2012**
(21) Anmeldenummer: 07726865.4
(22) Anmeldetag: 13.03.2007
(51) Int. Cl.: A23L 1/0524, A23L 1/068, A23L 1/064, A23L 1/05, A61K 47/36, A61K 9/00

(54) **VERFAHREN ZUR HERSTELLUNG EINES NAHRUNGS-, NAHRUNGSERGÄNZUNGS- ODER GENUSSMITTELS**
METHOD FOR THE PRODUCTION OF A FOOD, A FOOD SUPPLEMENT, OR CONSUMABLE GOOD
PROCÉDÉ DE PRODUCTION D'UN PRODUIT ALIMENTAIRE, D'UN COMPLÉMENT ALIMENTAIRE OU D'UNE FRIANDISE

(43) Veröffentlichungstag der Anmeldung: 27.01.2010
(73) Patentinhaber: Vymazal, Kurt, 3012 Wolfsgraben (AT)
(72) Erfinder: Vymazal, Kurt, 3012 Wolfsgraben (AT)
(74) Vertreter: Kruspig, Volkmar
(86) Internationale Anmeldenummer: PCT/EP2007/052364
(87) Internationale Veröffentlichungsnummer: WO 2008/110208

(56) Entgegenhaltungen:
- EP-A- 0 575 179
- EP-A- 0 930 017
- EP-A- 1 293 132
- WO-A-01/05248
- WO-A-02/071871
- US-A1- 2003 207 015
- US-B1- 6 699 977
- DATABASE WPI Week 200580 Derwent Publications Ltd., London, GB; AN 2005-783236 XP002463625 & JP 2005 325081 A (MEDREX KK) 24. November 2005 (2005-11-24)

## Beschreibung

Die Erfindung betrifft ein Verfahren zur Herstellung eines Nahrungs-, Nahrungsergänzungs- oder Genussmittels, wobei dieses eine Mischung aus wasserlöslichen Kohlenhydraten, Pektin, Wasser, Ca⁺⁺-Ionen sowie mindestens einem Verdickungsmittel enthält, gemäß Oberbegriff des Patentanspruchs 1.

Es ist bekannt, nicht formhaltige Gele in Tuben oder tubenähnlichen Behältnissen herzustellen, wobei die derartigen Gele zum Verzehr geeignet sind und als Nahrungsergänzungs- oder Genussmittel Verwendung finden. Üblicherweise werden nicht formhaltige Gele in Kunststoffbeuteln mit Aufreiß-Perforation oder zum Aufschneiden abgefüllt und angeboten und sind nicht oder nicht hygienisch einwandfrei wieder verschließbar. Ein Verzehr erfolgt hier nur durch direkten Kontakt mit dem Mund durch gleichzeitiges Drücken und Aussaugen oder durch Zuhilfenahme eines Löffels oder dergleichen Hilfsmittels.

Bekannt sind darüber hinaus auch Aluminiumtuben mit Schraubverschluss, z.B. zur Aufnahme von Senf, Mayonnaise, aber auch von Marmeladen. Bei derartigen Tuben mit Schraubverschluss ist ein Verschließen mit anschließender Wiederverwendung möglich. Durch den Verschluss wird eine Veränderung des Inhalts z.B. durch Einfluss von Luftsauerstoff vermieden.

Die vorerwähnten Verpackungsmittel enthalten als Füllgut Fruchtpasten, hoch- oder niedrigkalorische Fruchtaufstriche oder Gemüsepasten oder ähnliche pastöse Substanzen.

Nahrungs-, Nahrungsergänzungs- oder Genussmittel, welche eine Mischung aus wasserlöslichen Kohlenhydraten, Pektin, Wasser, Ca⁺⁺-Ionen sowie mindestens einem Verdickungsmittel enthalten, wobei der Mischung beispielsweise Fruchtsaft- und/oder Gemüsesaft-Konzentrate und/oder Alkohol zugegeben ist, sind u. a. aus EP 1 293 132 A1, WO 01/05248 A1 und EP 0 575 179 A2 bekannt.

Aus der WO 2002/0718171 A1 ist ein Verfahren zum Herstellen eines kalorienreduzierten Fruchtprodukts vorbekannt, wobei die Aufbereitung des Fruchtmaterials über eine intensive Vermischung von Einzelbestandteilen erfolgt, die unter anderem Pektin, Früchte und Süssungsmittel umfassen. Bei derartigen Mischungen ist eine Temperaturbehandlung zum Zweck der Pasteurisation zwingend erforderlich.

US 2003/207015 A1 offenbart ein Verfahren zur Herstellung von Fruchtpasten basierend auf Pektin, Wasser, Verdickungsmittel und Kohlehydraten durch Herstellung einer flüssigen Phase mit gelöstem Pektin, welche nicht geliert ist. Der Erhalt der Fruchtpaste erfolgt durch eine flüssige und eine trockene Phase. Am Ende des Verfahrens zur Herstellung der flüssigen Phase wird Fruchtpulver hinzugegeben. Die erwähnte trockene Phase enthält eine Kalziumquelle, Buffersalz und einen Stabilisator, welche zur flüssigen Phase gegeben wird.

Gelzusammensetzungen, welche Gellangummi und Pektin enthalten sind beispielsweise aus der EP 0 930 017 A1 bekannt. Gemäß der darin offenbarten Lehre werden zunächst Gellangummi, Pektin, Zucker und Wasser bei 85 °C für etwa 10 Minuten erhitzt und konzentrierter Fruchtsaft zugegeben. Es folgt eine Zugabe von Kalzium in Form von Kalziumacetat.

Zum Stand der Technik sei darüber hinaus noch auf die EP 0575179 A2 und die DE 10209394 A1 aufmerksam gemacht.

Aus dem Vorgenannten ist es Aufgabe der Erfindung, ein Verfahren zur Herstellung eines halbfesten, formhaltigen Gels anzugeben, das aus wieder verschließbaren Behältnissen entnommen werden kann und zum unmittelbaren Verzehr für Mensch und Tier geeignet ist. Eine Verabreichung soll ohne Hilfsmittel, wie z.B. Löffel oder dergleichen möglich sein.

Das Nahrungs-, Nahrungsergänzungsmittel soll zumindest gesundheitsfördernd sein, das Genussmittel soll Genusszwecken dienen und über eine lange Haltbarkeit verfügen, und zwar ohne dass aufwendige substanzzerstörende Temperaturbehandlungsschritte notwendig werden.

Auch bei wiederholter Auspressung von kleinen oder größeren Mengen des zu erhaltenden Gelprodukts, z.B. aus einer Tube, soll die gelartige Konsistenz erhalten bleiben, d.h. es ist eine unerwünschte Synärese zu unterbinden.

Die Lösung der Aufgabe der Erfindung erfolgt mit einem Verfahren gemäß Definition nach Patentanspruch 1, wobei die Unteransprüche mindestens zweckmäßige Ausgestaltungen und Weiterbildungen beinhalten.

Demnach wird bei dem Verfahren zur Herstellung des Nahrungs-, Nahrungsergänzungs- oder Genussmittels von einer Mischung ausgegangen, die wasserlösliche Kohlenhydrate, Pektin, Wasser, Ca⁺⁺-Ionen sowie mindestens ein Verdickungsmittel enthält.

Erfindungsgemäß werden der Mischung nach Erwärmen bis zur klaren Lösung des Pektins Fruchtsaft- und/oder Gemüsesaft-Konzentrate und/oder Alkohol zugegeben, wobei in einem Behältnis, insbesondere in eine Tube abgefüllt, ein durch Gelieren verzehrfertiges, formhaltiges Produkt entsteht.

Die der Mischung zugebbaren Fruchtsaft- und/oder Gemüsesaft-Konzentrate werden aus natürlich gewonnenen Säften durch Wasserentzug bereitgestellt.

Die Fruchtsaft- und/oder Gemüsesaft-Konzentrate werden nach Lösen der Gelbildner der Mischung zugesetzt, so dass die Temperaturbelastung bezogen auf die Fruchtsaft- oder Gemüsesaft-Konzentrate gering gehalten werden kann.

Zum Vermeiden einer unerwünschten Phasentrennung beim Einbringen des Alkohols in die Mischung wird der Alkohol in unmittelbarer Nähe einer Kombination von hochtourig laufenden, in der Mischung eingetauchten Pürierstäben zugeführt, so dass die gewünschte innige Durchmischung der gelierenden Lösung erfolgt.

Die Kombination wie vorerwähnt umfasst mindestens zwei in einem Winkel zueinander stehende Pürierstäbe, wobei sich durch die Winkelstellung der Pürierstäbe zueinander bei gleicher Drehrichtung im Mischungsbereich eine die Durchmischung extrem fördernde Gegenläufigkeit ergibt.

Der Mischung können bis zu 30 Gew.-% Alkohol unter Nutzung der verfahrensgemäßen Lehre zugegeben werden, was ansonsten aufgrund der Phasentrennung zwischen dem Alkohol und dem pektinhaltigen Gel unmöglich ist.

Bei alkoholfreien Gelprodukten mit Fruchtsaft-Konzentraten wird zum Zweck der Verbesserung der Haltbarkeit Ascorbinsäure und/oder Sorbinsäure und/oder deren Salze der Mischung zugegeben.

Bei eingesetzten Gemüsesaft-Konzentraten insbesondere aus Erdgemüse erfolgt eine Autoklavierung, und zwar hier in einem bestimmten Temperaturbereich von im Wesentlichen 120°C über eine Zeitdauer von mindestens 15 Minuten.

Wenn notwendig, können der Mischung mit Gemüsesaft-Konzentraten aus Erdgemüse zusätzlich Konservierungsmittel zugegeben werden.

Das Abfüllen des formhaltigen Gelprodukts erfolgt in einem Behältnis, welches aus einem nicht elastischen Material besteht, um beim Ausdrücken oder Auspressen des Gelprodukts aus dem Behältnis eine hin- und hergehende Bewegung des Gels und damit eine Synärese zu vermeiden. Bevorzugt besteht hier das Behältnis aus einer Aluminiumtube der an sich bekannten Art.

Die Mischung kann darüber hinaus Vitamine, Coffein, Taurin und/oder Mehrfachkonzentrate von Spurenelementen enthalten.

Wenn das Behältnis eine metallische Tube ist, besteht die Möglichkeit, mittels Auspressen über die so erhaltene Gel-Stranglänge eine Abschätzung der Produktmenge zum Zweck der Dosierung vorzunehmen.

Die Erfindung soll nachstehend anhand eines Ausführungsbeispiels näher erläutert werden.

Beim Ausführungsbeispiel gilt es, ein halbfestes, formhaltiges Gel herzustellen, das aus einer wieder verschließbaren Tube oder aus einer Injektionsspritze ohne Nadel drück- oder pressbar ist, so dass ein unmittelbarer Verzehr für Mensch oder Tier erfolgen kann. Durch bloßes Abmessen oder Abschätzen der Länge des ausgepressten oder ausgedrückten Gel-Strangs besteht die Möglichkeit einer näherungsweisen dosierten Verabreichung der Inhaltsstoffe. Hier sind weitere Hilfsmittel nicht notwendig.

Durch den Einsatz von Behältnissen in Form von Injektionsspritzen ähnlichen Gebilden mit Skalierung ohne Nadeleinsatz ist eine exakte Dosierung möglich, wenn dies applikativ beabsichtigt ist.

Das erfindungsgemäß realisierbare Nahrungs-, Nahrungsergänzungs- oder Genussmittel oder aber auch Ergänzungsfuttermittel kann alkoholhaltig oder alkoholfrei ausgeführt werden und basiert auf Pektinbasis. Auf jeden Fall ist durch die gelartige Konsistenz beim Ausdrücken oder Auspressen sichergestellt, dass das Produkt einen formhaltigen Strang bildet. Auch beim Auspressen des Stranges auf z.B. den Handrücken bleibt trotz Einwirkung der Körpertemperatur die Gelkonsistenz verzehroptimal erhalten. d.h. es findet kein Wegfließen des Produkts statt.

Das Produkt entsteht durch Mischen von z.B. Apfel- oder Citruspektin, amidiert oder nicht amidiert mit wasserlöslichen Kohlenhydraten, Wasser, Ca⁺⁺-Ionen und Verdickungsmitteln wie Carrageen und Johannisbrotkernmehl oder Agar Agar. Die Mischung wird bis zum Erhalt einer klaren Lösung des Pektins erwärmt. Hiernach erfolgt ein Zusatz von Fruchtsaft- oder Gemüsesaft-Konzentraten und/oder Alkohol. Nach Abfüllung in bevorzugt Aluminiumtuben oder ähnliche Behältnisse bildet sich durch Gelierung das formhaltige Gel.

Nach Art des verwendeten Pektins wird der pH-Wert der Lösung im leicht sauren Milieu von etwa pH 3,0 bis 3,5 gehalten.

Der Mischung können Vitamine, Mineralstoffe, Spurenelemente, Aromen, Farbstoffe und andere Substanzen wie Coffein, Taurin, Glucose und/oder andere Lebensmittel-Zusatzstoffe, wie z.B. Konservierungsmittel zugesetzt werden, um hier verschiedene Sorten des Gels entstehen zu lassen.

Bei einer ersten Ausführungsform der Erfindung werden Fruchtsaft- und Gemüsesaft-Konzentrate zur Herstellung des formhaltigen Gels für Genuss - und/oder Ernährungszwecke verwendet.

Bei einer zweiten Ausführungsform der Erfindung erfolgt der Einsatz von Alkohol unter Berücksichtigung eines speziellen Verfahrens zur Inkorporierung des Alkohols in das Gel.

Die Fruchtsaft- oder Gemüsesaft-Konzentrate werden aus natürlich gepressten oder gewonnenen Frucht- und Gemüsesäften durch schonenden Entzug von Wasser produziert. Hierdurch bleiben sämtliche Inhaltsstoffe des Saftes sowie natürliche Aromen und Farbstoffe erhalten.

Durch den Zusatz der Konzentrate nach dem Lösen der Gelbildner durch Kochen werden die Fruchtsaft- oder Gemüsesaft-Konzentrate nur kurz einer hohen Temperatur von im Wesentlichen 95°C ausgesetzt, so dass Farbe und Aroma erhalten bleiben.

Es hat sich gezeigt, dass Alkohol sich nur sehr schwer mit bekannten Methoden wie Rühren in pektinhaltiges Gel einmischen lässt, da die Phasen getrennt bleiben und Synärese auftritt.

Erfindungsgemäß wird jedoch der Alkohol bei einer Ausführungsform durch ein Rohr, das unmittelbar über den Propellerblättern eines hochtourig laufenden Pürierstabes, der in der Lösung eingetaucht läuft, endet, zugeführt, so dass die gewünschte optimale Einbindung des Alkohols in das Gel erreichbar ist.

Dabei hat sich gezeigt, dass es von besonderem Vorteil ist, wenn in unmittelbarer Nähe eines ersten Pürierstabes gegenüberliegend in einem Winkel von ca. 120° ein zweiter hochtouriger Pürierstab angeordnet ist, so dass eine innige Durchmischung der gelierenden Lösung erfolgt. Da beide Pürierstäbe die gleiche Drehrichtung haben, ergibt sich bei schräger Anordnung ein gegenläufiger Betrieb. Durch dieses Verfahren wird eine gewünschte ansprechende haptische Qualität, ausreichende Homgenität und stabile Textur erreicht. Durch das vorgestellte Verfahren können Alkoholgehalte bis zu 30 Gew.-% bezogen auf die Mischung realisiert werden.

Da die eingesetzten Rohstoffe keimarm sind und der pH-Wert im Bereich von 3,0 bis 3,5 liegt, ist unter Anwendung der erläuterten Methode an sich eine weitere Konservierung nicht erforderlich, um eine ausreichende Haltbarkeit zu erzielen.

Bei alkoholfreien Gelen mit Fruchtsaft-Konzentraten als Grundstoff ist der Zusatz von Konservierungsmitteln wie Ascorbinsäure und/oder Sorbinsäure und/oder deren Salze empfehlenswert.

Bei alkoholfreien Gelen mit Gemüsesaft-Konzentraten aus Erdgemüse, wie z.B. Karotten als Grundstoff, ist neben dem Zusatz von Konservierungsmitteln wie Ascorbinsäure und/oder Sorbinsäure und/oder deren Salze eine Autoklavierung bei im Wesentlichen 120°C und einer Behandlungsdauer von ca. 15 Minuten angebracht.

Aufgrund der nicht elastischen bzw. starren Wand des eingesetzten Behältnisses wird das Gel beim Ausdrücken oder Auspressen nicht unerwünscht hin- und herbewegt, sondern immer nur in eine Richtung orientiert. Hierdurch bleibt die Textur des Gels erhalten und eine Synärese tritt nicht auf.

Da die Stärke und damit Dicke des formhaltigen Gel-Strangs gleichbleibend ist, kann eine dosierte Verabreichung von Inhaltsstoffen erfolgen, wenn die Zweckbestimmung der Produkte Nahrungsergänzung oder Arzneimittel ist.

## Patentansprüche

1. Verfahren zur Herstellung eines Nahrungs-, Nahrungsergänzungs- oder Genussmittels, wobei dieses eine Mischung aus wasserlöslichen
Kohlenhydraten, Pektin, Wasser, Ca⁺⁺-Ionen sowie mindestens ein Verdickungsmittel enthält,
**dadurch gekennzeichnet, dass**
der Mischung nach Erwärmen bis zur klaren Lösung des Pektins Fruchtsaft - und/oder Gemüsesaft-Konzentrate und/oder Alkohol zugegeben sind, wobei in ein Behältnis, insbesondere eine Tube abgefüllt, ein durch Gelieren verzehrfertiges form haltiges Produkt entsteht.

2. Verfahren nach Anspruch 1,
**dadurch gekennzeichnet, dass**
die der Mischung zugegebenen Fruchtsaft- und/oder Gemüsesaft-Konzentrate aus natürlich gewonnenen Säften durch Wasserentzug bereitgestellt werden.

3. Verfahren nach Anspruch 1 oder 2,
**dadurch gekennzeichnet, dass**
die Fruchtsaft- und/oder Gemüsesaft-Konzentrate nach Lösen der Gelbildner der Mischung zugesetzt werden, um deren Temperaturbelastung gering zu halten.

4. Verfahren nach einem der vorangegangenen Ansprüche,
**dadurch gekennzeichnet, dass**
zum Vermeiden einer Phasentrennung beim Einbringen des Alkohols in die Mischung der Alkohol in unmittelbarer Nähe einer Kombination von hochtourig laufenden, in der Mischung eingetauchten Pürierstäben zugeführt wird, so dass eine innige Durchmischung der gelierenden Lösung erfolgt.

5. Verfahren nach Anspruch 4,
**dadurch gekennzeichnet, dass**
die Kombination mindestens zwei, in einem Winkel zueinander stehende Pürierstäbe umfasst, wobei sich durch die Winkelstellung der Pürierstäbe zueinander bei gleicher Drehrichtung im Mischungsbereich eine die Durchmischung fördernde Gegenläufigkeit ergibt.

6. Verfahren nach einem der vorangegangenen Ansprüche,
**dadurch gekennzeichnet, dass**
der Mischung bis zu 30 Gew.-% Alkohol zugegeben werden.

7. Verfahren nach einem der vorangegangenen Ansprüche,
**dadurch gekennzeichnet, dass**
bei alkoholfreien Gelprodukten mit Fruchtsaft-Konzentraten Ascorbinsäure und/oder Sorbinsäure und/oder deren Salze der Mischung zugegeben werden.

8. Verfahren nach einem der vorangegangenen Ansprüche, **dadurch gekennzeichnet, dass**
bei alkoholfreien Gelprodukten wie Gemüsesaft-Konzentraten aus Erdgemüse eine Autoklavierung erfolgt.

9. Verfahren nach Anspruch 8,
**dadurch gekennzeichnet, dass**
der Mischung Konservierungsmittel zugegeben werden.

10. Verfahren nach einem der vorangegangenen Ansprüche,
**dadurch gekennzeichnet, dass**
das Abfüllen des formhaltigen Produkts in ein Behältnis erfolgt, welches aus einem nicht elastischen Material besteht, um beim Ausdrücken oder Auspressen des Gelprodukts aus dem Behältnis eine hin- und hergehende Bewegung des Gels und damit eine Synärese zu vermeiden oder zu unterdrücken.

11. Verfahren nach einem der vorangegangenen Ansprüche,
**dadurch gekennzeichnet, dass**
der Mischung Vitamine, Coffein, Taurin und/oder Mehrfachkonzentrate von
Spurenelementen und/oder Mineralstoffen zugegeben wird.

12. Verfahren nach einem der vorangegangenen Ansprüche,
**dadurch gekennzeichnet, dass**
das Behältnis eine metallische Tube ist, wobei mittels Auspressen über die so erhaltene Gel-Stranglänge eine Abschätzung der Produktmenge zum Zweck der Dosierung erfolgt.

## Claims

1. Method for producing a foodstuff, a food supplement or luxury food, which contains a mixture of water-soluble carbohydrates, pectin, water, Ca⁺⁺ ions as well as at least one thickening agent,
**characterized in that**
the mixture, after heating it to obtain a clear solution of the pectin, is added fruit juice and/or vegetable juice concentrates and/or alcohol so as to obtain by means of gelling a product ready for consumption and with a stable shape when filled into a container, especially into a tube.

2. Method according to claim 1,
**characterized in that**
the fruit juice and/or vegetable juice concentrates added to the mixture are provided from naturally obtained juices by means of water extraction.

3. Method according to claim 1 or 2,
**characterized in that**
the fruit juice and/or vegetable juice concentrates are added to the mixture after the gelling agents are dissolved so as to keep the thermal stress on them low.

4. Method according to one of the preceding claims,
**characterized in that**
in order to avoid a phase separation when adding the alcohol to the mixture, the alcohol is added in the direct proximity of a combination of immersion blenders running at high speed and immersed into the mixture, so that a homogenous intermixing of the gelling solution takes place.

5. Method according to claim 4,
**characterized in that**
the combination comprises at least two immersion blenders positioned at an angle relative to one another, wherein the angular position of the immersion blenders relative to one another, with the same sense of rotation in the mixing area, results in a contrarotation which enhances the intermixing.

6. Method according to one of the preceding claims,
**characterized in that**
up to 30 percent by weight of alcohol are added to the mixture.

7. Method according to one of the preceding claims,
**characterized in that**
for nonalcoholic gel products containing fruit juice concentrates ascorbic acid and/or sorbic acid and/or the salts thereof are added to the mixture.

8. Method according to one of the preceding claims,
**characterized in that**
for nonalcoholic gel products such as vegetable juice concentrates from earth grown vegetables an autoclaving is performed.

9. Method according to claim 8,
**characterized in that**
preservatives are added to the mixture.

10. Method according to one of the preceding claims,
**characterized in that**
the product, which has a stable shape, is filled into a container formed of a non-elastic material so as to avoid or suppress a back and forward motion of the gel and, thus, a syneresis when the gel product is squeezed or pressed out of the container.

11. Method according to one of the preceding claims,
**characterized in that**
vitamins, caffeine, taurine and/or multiple concentrates of trace elements and/or mineral substances are added to the mixuture.

12. Method according to one of the preceding claims,
**characterized in that**
the container is a metallic tube, wherein by means of the length of the gel stripe obtained by pressing out an estimation of the amount of the product is made for dosing purposes.

## Revendications

1. Procédé pour la production d'un produit alimentaire, d'un complément alimentaire ou d'un aliment d'agrément, celui-ci contenant un mélange d'hydrocarbures solubles dans l'eau, de pectine, d'eau, d'ions Ca++, ainsi qu'au moins un agent épaississant,
**caractérisé en ce que**, après chauffage jusqu'à dissolution claire de la pectine, des concentrés de jus de fruits et/ou de jus de légumes et/ou de l'alcool sont ajoutés au mélange, de sorte qu'il se forme, une fois rempli dans un récipient, en particulier dans un tube, un produit conservant sa forme par gélification et prêt à la consommation.

2. Procédé selon la revendication 1,
**caractérisé en ce que** les concentrés de jus de fruits et/ou de jus de légumes ajoutés au mélange sont préparés par déshydratation à partir de jus obtenus de façon naturelle.

3. Procédé selon la revendication 1 ou 2,
**caractérisé en ce que** les concentrés de jus de fruits et/ou de jus de légumes sont ajoutés au mélange après dissolution de l'agent de gélification afin de maintenir faible leur sollicitation en température.

4. Procédé selon l'une des revendications précédentes,
**caractérisé en ce que**, pour éviter une séparation de phase lors de l'introduction de l'alcool dans le mélange, l'alcool est ajouté au voisinage immédiat d'une combinaison de barreaux de malaxage qui plongent dans le mélange et qui tournent à haute vitesse, de sorte qu'il se produit un mélange intime de la solution en cours de gélification.

5. Procédé selon la revendication 4,
**caractérisé en ce que** la combinaison comprend au moins deux barreaux de malaxage disposés sous un angle l'un par rapport à l'autre, de sorte qu'il en résulte, du fait de la position angulaire des barreaux de malaxage l'un par rapport à l'autre et dans le même sens de rotation, un déplacement en sens contraire qui favorise la formation du mélange dans la zone de mélange.

6. Procédé selon l'une des revendications précédentes,
**caractérisé en ce que** l'on ajoute au mélange jusqu'à 30 % en poids d'alcool.

7. Procédé selon l'une des revendications précédentes,
**caractérisé en ce que** pour des produits gélifiés sans alcool avec des concentrés de jus de fruits, en ajoute au mélange de l'acide ascorbique et/ou de l'acide sorbique et/ou des sels de ces acides.

8. Procédé selon l'une des revendications précédentes,
**caractérisé en ce que** pour des produits gélifiés sans alcool comme des concentrés de jus de légumes provenant de légumes poussant en terre, on procède à un traitement à l'autoclave.

9. Procédé selon la revendication 8,
**caractérisé en ce que** l'on ajoute des agents de conservation au mélange.

10. Procédé selon l'une des revendications précédentes,
**caractérisé en ce que** le remplissage du produit conservant sa forme a lieu dans un récipient qui est en un matériau non élastique afin d'éviter ou de supprimer, lors de l'enfoncement ou du pressage du produit gélifié hors du récipient, un mouvement en va-et-vient du gel et ainsi une synérèse.

11. Procédé selon l'une des revendications précédentes,
**caractérisé en ce que** l'on ajoute au mélange des vitamines, de la caféine, de la taurine et/ou des concentrés multiples d'oligo-éléments et/ou de produits minéraux.

12. Procédé selon l'une des revendications précédentes,
**caractérisé en ce que** le récipient est un tube métallique, et au moyen du pressage via la longueur du cordon de gel ainsi obtenu on dispose d'une estimation de la quantité de produit en vue du dosage.
